# EUROPEAN PATENT APPLICATION

(11) **EP 2 128 166 A1**
(43) Date of publication of application: **02.12.2009**
(21) Application number: 08156592.1
(22) Date of filing: 20.05.2008
(51) Int. Cl.: C07F 9/38, A61P 35/00

(54) **Polymorphic forms of Ibandronate sodium and processes for preparation thereof**

(71) Applicant: Chemo Ibérica, S.A., 08028 Barcelona (ES)
(72) Inventor: Ventimiglia, Gianpiero, 72021, Francavilla Fontana (BR) (IT); Magrone, Domenico, 20128, Milano (IT); Castaldi, Graziano, 28072, Briona (NO) (IT)
(74) Representative: Barlocci, Anna

(57) **Abstract**

Polymorphic crystalline forms of Ibandronate sodium were found, referred to hereinafter as polymorphic Form α, Form β, Form ε. Form δ and Form γ. Furthermore, the present invention is directed to processes for the preparation of these crystalline forms, to pharmaceutical compositions comprising them, as well as their use.

## Description

### Field of the invention

The present invention relates to novel polymorphic forms of Ibandronate sodium, to processes for preparing them, to pharmaceutical compositions containing them, as well as their use.

### Background of the invention

Ibandronate sodium is a third-generation nitrogen-containing biphosphonate derivative characterized by an aliphatic tertiary amine side chain.
Ibandronate sodium is one of the most potent anti-resorptive drugs that directly inhibit osteoclast activity, presenting a pharmacologic alternative for controlling hypercalcemia. Ibandronate sodium binds to hydroxyapatite in calcified bones, rendering them resistant to hydrolytic dissolution by phosphatases, thereby inhibiting both normal and abnormal bone resorption. This drug increases bone mass and decreases the risk of fractures and is therefore particularly adapted to bone diseases and calcium metabolic disorders such as osteoporosis, osteolysis, Paget's disease, Bechterew's disease, bone metastases, urolithiasis, heterotropic ossifications, rheumatoid arthritis, osteoarthritis and degenerative arthrosis.
Ibandronate sodium is a compound of Formula (1) 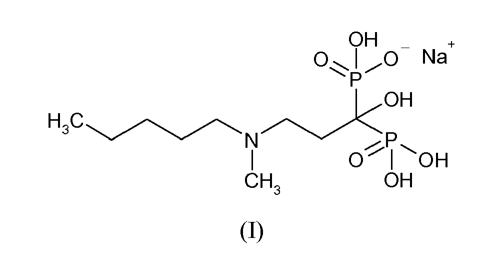 [3-(N-methyl-N'-pentyl)-amino-1-hydroxypropane-1,1-diphosphonic acid monosodium salt], which is disclosed in U.S. Patent 4,927,814. It is marketed under the name of Boniva® for the treatment of bone disorders such as hypercalcaemia of malignancy, osteolysis, Paget's disease, osteoporosis, and metastatic bone disease. Ibandronate sodium is also marketed in Europe under the name of Bondronat®.

The present invention relates to novel polymorphic forms of Ibandronate sodium and processes for preparation thereof.

Polymorphism is the property of some molecules and molecular complexes to assume more than one crystalline or amorphous form in the solid state. Substances are known which only appear in a single crystal form; in addition, however, there are also substances which can form two, three or even more polymorphic crystal modifications. In general, polymorphism is caused by the ability of the molecule of a substance to change its conformation or to form different inter molecular and intramolecular interactions, particularly hydrogen bonds, which is reflected in different atom arrangements in the crystal lattices of different polymorphs. Accordingly, polymorphs are distinct solids sharing the same molecular Formula, having distinct advantageous and/or disadvantageous physical properties compared to other forms in the polymorph family.

The morphology and polymorphology of organo-chemical active substances is of great importance to the chemical and pharmaceutical development thereof.

The relevant polymorphism of an organo-chemical substance is always unpredictable in respect of the number of crystal modifications, the stability thereof and their behaviour in a living organism.

The different polymorphs of a substance possess different energies of the crystal lattice and, thus, they show different physical properties of the solid state such as form, density, melting point, colour, stability, dissolution rate, milling facility, granulation, compacting etc. These differences in morphology and polymorphism may have drastic effects on the flowability of the milled solid (flowability affects the ease with which the material is handled during processing into a pharmaceutical product; when particles of the powdered compound do not flow past each other easily, a formulation specialist must necessitate the use of glidants), development, transport stability and storage stability of individual administration forms, on the ability to produce different administration forms, on their application, on the solubility in polar or non-polar, protic or aprotic solvents, on solubility in aqueous solution, on solubility in the gastric juices, on solubility in blood serum, and finally on bio-availability. The rate of dissolution of an active ingredient in a patient's stomach fluid can have therapeutic consequences since it imposes an upper limit on the rate at which an orally-administered active ingredient can reach the patient's bloodstream. The rate of dissolution is also a consideration in formulating syrups, elixirs and other liquid medicaments. Other important properties of polymorphic forms relate to the ease of processing the form into pharmaceutical dosages, as the tendency of a powdered or granulated form to flow and the surface properties that determine whether crystals of the form will adhere to each other when compacted into a tablet. The polymorphic form may give rise to thermal behavior different from that of the amorphous material or another polymorphic form. Thermal behavior is measured in the laboratory by such techniques as capillary melting point, Differential Scanning Calorimetry (DSC) and can be used to distinguish some polymorphic forms from others. A particular polymorphic form may also give rise to distinct spectroscopic properties that may be detectable by X-Ray Powder Diffraction (XRPD).

Generally, the crystalline solid has improved chemical and physical stability over the amorphous form, and forms with low crystallinity. They can also exhibit improved solubility, hygroscopicity, bulk properties, and/or flowability. The same also applies in respect of the physical and chemical properties of Ibandronate sodium.

It has been found that Ibandronate sodium may exist in various polymorphs.

According to the Patent Applications WO-A-2006081962 and WO-A-2006081963, two crystalline form of Ibandronate sodium, monohydrate salt, herein designated as Forms A and B, were disclosed.

The Patent Application WO-A-2006024024 relates to solid amorphous and crystalline forms of Ibandronate sodium (herein designated as crystalline Forms C, D, E, F, G, H, J, K, K2, K3, Q, Q1, Q2, Q3, Q4, Q5, Q6, QQ, R, S, T) and processes for preparation thereof, where some forms of them are solvates. Form C can exist as monoethanolate, Form E can exist as hemibutanolate, Form R and S can exist as hemiethanolate.

The Patent Application WO-A-2007074475 relates to novel polymorphic forms of Ibandronate sodium monohydrate and processes for their preparation. The novel crystalline forms are designated as Form I and Form II, and the amorphous Ibandronate sodium monohydrate as Form III.

The discovery of novel polymorphic forms of a pharmaceutically useful compound provides a new opportunity to improve the performance characteristics of a pharmaceutical product. It enlarges the repertoire of materials that a formulation scientist has available for designing a pharmaceutical dosage form, or a drug with a targeted release profile, or other desired characteristics, such as flowability and suitable rate of dissolution in aqueous fluid. Therefore, the preparation of novel crystalline forms of Ibandronate sodium is desirable.

An object of the present invention is to provide novel polymorphic crystalline forms of Ibandronate sodium, processes for preparing them, pharmaceutical compositions containing them, as well as their use.

### Summary of the invention

The present invention provides Ibandronate sodium in new crystalline forms designated as Form α, Form β, Form ε, Form δ and Form γ.

In one aspect, the invention provides a novel polymorphic crystalline form of Ibandronate sodium, herein designated as Form α. The crystalline Form α of Ibandronate sodium is characterized by an XRPD pattern comprising peaks at about 5.0, 10.0, 16.1, 19.8, 27.7 ± 0.2° 2θ. Form α can exist as a solvate with N-methyl-2-pyrrolidinone.

In another aspect, the invention provides a novel polymorphic crystalline form of Ibandronate sodium, herein designated as Form β. The crystalline Form β of Ibandronate sodium is characterized by an XRPD pattern comprising peaks at about 4.9, 9.6, 17.1, 19.8 ± 0.2° 2 θ. Form β can exist as a solvate with 2-propanol.

In another aspect, the invention provides a novel polymorphic crystalline form of Ibandronate sodium, herein designated as Form ε. The crystalline Form ε of Ibandronate sodium is characterized by an XRPD pattern comprising peaks at about 4.9, 6.0, 17.1, 19.8 ± 0.2° 2 θ. Form ε can exist as a solvate with 2-propanol.

In another aspect, the invention provides a novel polymorphic crystalline form of Ibandronate sodium, herein designated as Form δ. The crystalline Form δ of Ibandronate sodium is characterized by an XRPD pattern comprising peaks at about 5.8, 17.0, 25.6, 26.3, 30.8 ± 0.2° 2 θ.

In another aspect, the invention provides a novel polymorphic crystalline form of Ibandronate sodium, herein designated as Form γ. The crystalline Form γ of Ibandronate sodium is characterized by an XRPD pattern comprising peaks at about 4.9, 6.1, 6.5, 12.2, 16.8, 25.8 ± 0.2° 2 θ.

Forms α, β and ε are stable solvate forms, allowing the isolation and purification of ibandronate sodium in high yields. Anhydrous Form δ and monohydrate Form γ are stable crystalline forms, which can be obtained from the corresponding solvate forms without degenerative phenomena. The new forms δ and γ, more in particular Form γ, resulted to be more useful for formulation. Solvates β and ε are useful for preparing said forms, respectively.

In another aspect, the invention provides a process for preparing the crystalline Form α of Ibandronate sodium comprising the steps of:
a) dissolving Ibandronate sodium in water;
b) heating the solution;
c) pouring the solution in N-methyl-2-pyrrolidinone to obtain a precipitate;
d) recovering the crystalline form.

In another aspect, the invention provides a process for preparing the crystalline Form β of Ibandronate sodium comprising the steps of:
a) dissolving ibandronate sodium in water;
b) heating the solution;
c) pouring the solution in 2-propanol to obtain a precipitate;
d) recovering the crystalline form.
   In another aspect, the invention provides a process for preparing the crystalline Form ε of Ibandronate sodium comprising the steps of:
e) suspending ibandronate sodium in a mixture of water and 2-propanol;
f) heating the suspension in order to obtain a clear solution;
g) cooling the solution in order to crystallize a solid;
h) recovering the crystalline form.

In another aspect, the invention provides a process for preparing the crystalline Form δ of Ibandronate sodium comprising the step of desolvating Ibandronate sodium Form β by heating the solid under reduced pressure.

In another aspect, the invention provides a process for preparing the crystalline Form γ of Ibandronate sodium comprising the step of desolvating Ibandronate sodium Form ε by heating the solid under reduced pressure.

In another aspect, the invention provides a crystalline Forms δ or γ of Ibandronate sodium for use as a medicament.

In another aspect, the invention provides a pharmaceutical composition comprising the crystalline Forms δ or γ of Ibandronate sodium, optionally together with at least one pharmaceutically acceptable excipient.

### Brief Description of the Drawings

Figure 1 provides an XRPD pattern of crystalline Form α of Ibandronate sodium.
Figure 2 provides an XRPD pattern of crystalline Forum β of Ibandronate sodium.
Figure 3 provides an XRPD pattern of crystalline Form ε of Ibandronate sodium.
Figure 4 provides an XRPD pattern of crystalline Form δ of Ibandronate sodium.
Figure 5 provides an XRPD pattern of crystalline Form γ of Ibandronate sodium.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The term "about" encompasses the range of experimental error that may typically occurs in a measurement.

The term "excipient" herein means any substance, not itself a therapeutic agent, used as a carrier or vehicle for delivery of a therapeutic agent to a subject or added to a pharmaceutical composition to improve its handling or storage properties or to permit or facilitate formation of a dose unit of the composition into a discrete article such as a tablet, capsule, pill, powder, granule, pellet, lozenge, pastille, elixir, syrup, solution, suspension, emulsion, drop, lotion, spray, tincture, cream, ointment, gel, unguent, suppository and transdermal devices for oral, enteral, parenteral or topical administrations.

The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient.

We have now surprisingly found that Ibandronate sodium may be prepared in a manner that result in novel polymorphic forms that are until now unknown. The novel polymorphic forms have been designated as Forms α, β, ε, δ, γ of Ibandronate sodium. These novel forms of Ibandronate sodium are found to be stable at room temperature, reproducible and suitable for pharmaceutical preparations. They can be prepared with efficient and economic processes particularly suited to large-scale preparation.

XRPD analysis was used to characterize polymorphic forms of Ibandronate sodium.

XRPD analysis was performed on a APD 2000 Ital Structures diffractometer at room temperature, using a CuKα tube (40 kV, 30 mA, λ = 1.5418 Å) as the X-ray source. Data collection was made in 2θ step scan mode, at a scan speed of 0.02°/s in the range of 3° to 40° in 2θ. Approximately 100 mg samples were accurately grinded and placed on an aluminium sampler.

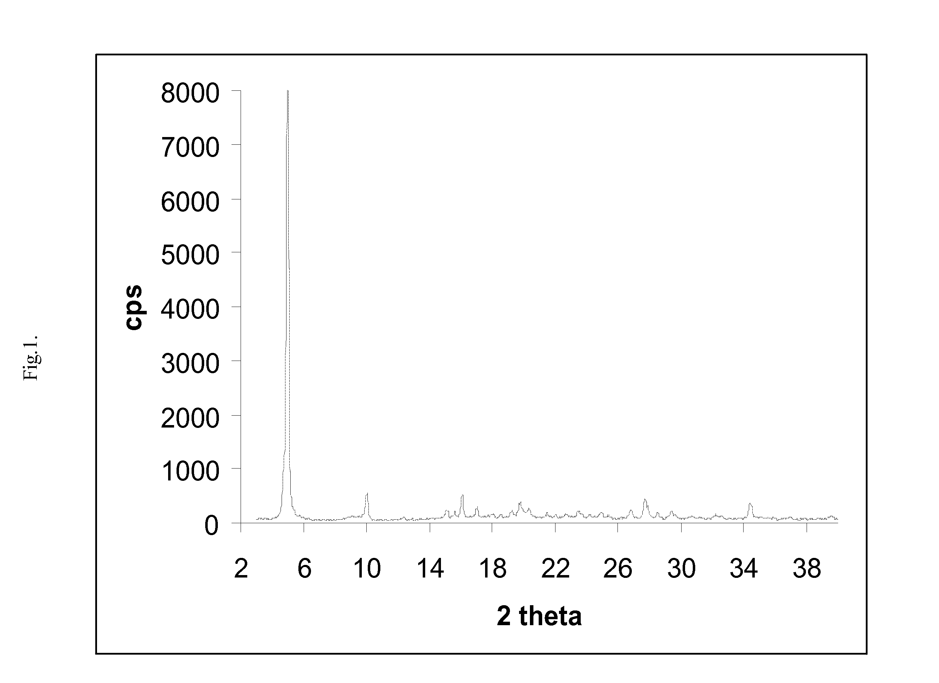

The novel crystalline Form α of Ibandronate sodium has an XRPD pattern as depicted in Figure 1.
The crystalline Form α of Ibandronate sodium can exist as solvate with N-methyl-2-pirrolidinone.

In another aspect, the invention provides a process for the preparation of Form α of Ibandronate sodium comprising the steps of:
a) dissolving Ibandronate sodium in water at a suitable temperature to dissolve Ibandronate sodium in water. Preferably, the temperature is comprised in a range of about 10 °C to about 30 °C; more preferably, the temperature is comprised in a range of about 15 °C to about 25 °C.
   Preferably, Ibandronate sodium is in a ratio of about 1:1 to about 1:1.5 weight/volume to water;
b) heating the solution in order to dissolve completely the Ibandronate sodium. Preferably, the solution is heated to a temperature comprised in a range of about 80 to about 100 °C;
c) pouring the solution in N-methyl-2-pyrrolidinone at a suitable temperature to obtain a precipitate. A suitable temperature is comprised in a range of about 5 °C to about 25 °C; preferably, the temperature is comprised in a range of about 10 °C to about 20 °C; more preferably, the temperature is about 15 °C. Preferably, Ibandronate sodium is in a ratio of about 1:5 to about 1:20 weight/volume with respect to N-methyl-2-pirrolidinone.
d) recovering the crystalline form.

The crystalline forms of the present invention may be recovered by methods well known to those skilled in the art for the separation of the crystallized solid from the mother liquor, for example by filtration, with or without the assistance of pressure and/or vacuum, or by centrifugation, or by decantation. The collected solid is washed with at least a solvent and dried by conventional means.

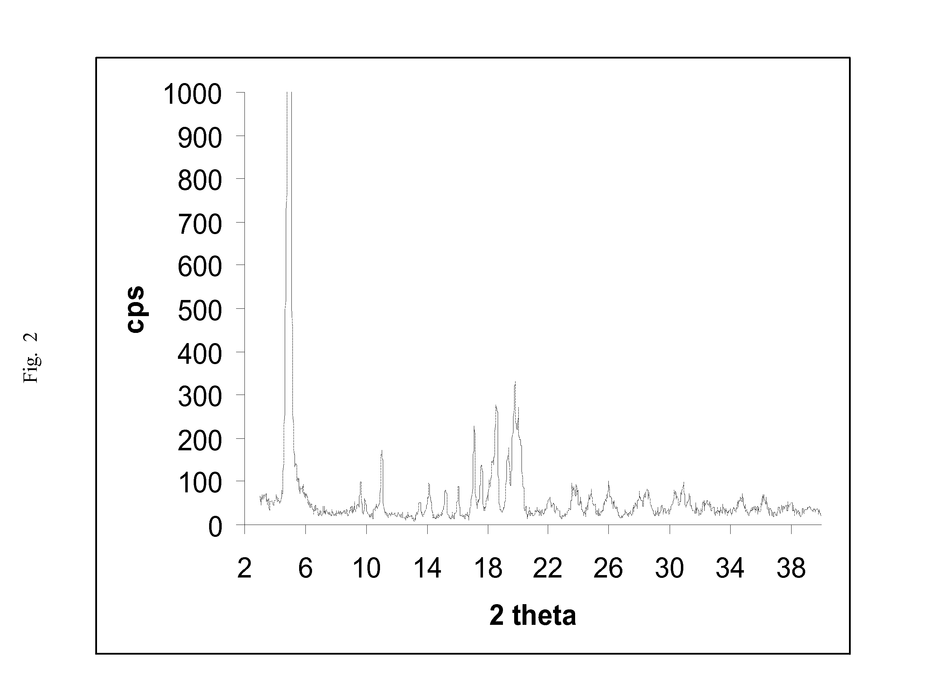

The novel crystalline Form β of Ibandronate sodium has an XRPD pattern as depicted in Figure 2.

In another aspect, the invention provides a process for the preparation of Form β of Ibandronate sodium comprising the steps of:
a) dissolving Ibandronate sodium in water at a suitable temperature. A suitable temperature is comprised in a range of about 10 °C to about 30 °C; preferably, the temperature is comprised in a range of about 15 °C to about 25 °C. Ibandronate sodium is in a ratio of about 1:1 to about 1:1.5 weight/volume to water.
b) heating the solution heating the solution in order to dissolve completely the Ibandronate sodium. Preferably, the solution is heated to a temperature comprised in a range of about 80 to about 100 °C;
c) pouring the solution in 2-propanol at a suitable temperature to obtain a precipitate. A suitable temperature is comprised in a range of about 5 °C to about 25 °C; preferably, the temperature is comprised in a range of about 10 °C to about 20 °C; more preferably, the temperature is about 15 °C. Preferably, Ibandronate sodium is in a ratio of about 1:5 to about 1:20 weight/volume with respect to 2-propanol.
d) recovering the crystalline form.

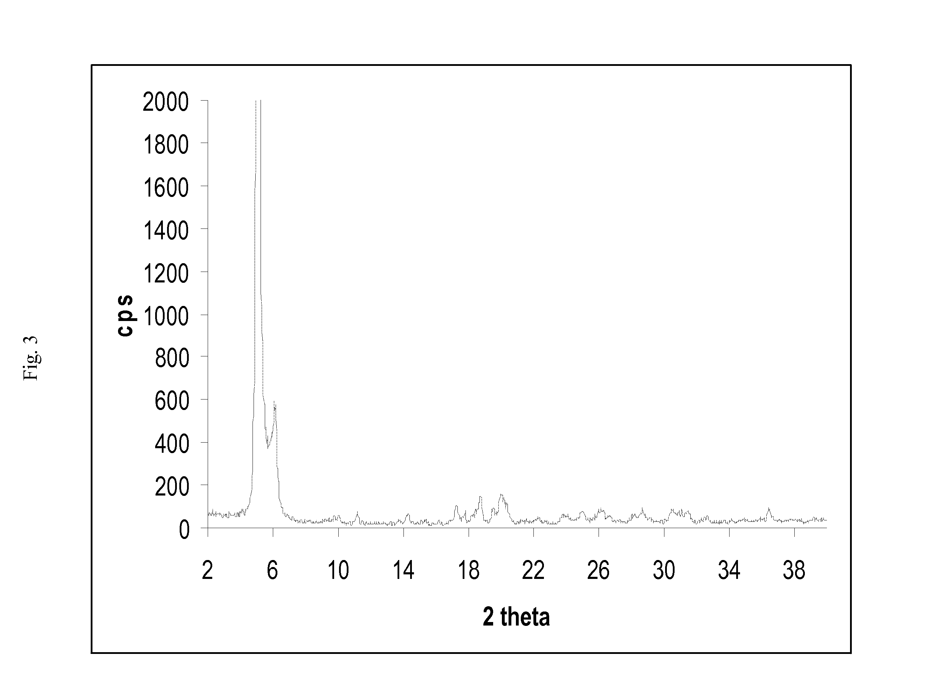

The novel crystalline Form ε of Ibandronate sodium has an XRPD pattern as depicted in Figure 3.

In another aspect, the invention provides a process for the preparation of Form ε of Ibandronate sodium comprising the steps of:
a) suspending Ibandronate sodium in a mixture of water and 2-propanol. Preferably, water and 2-propanol are in a ratio of about 1:3 to about 1:5 volume/volume. Preferably, Ibandronate sodium is in a ratio of about 1:4 to about 1:6 weight/volume with respect to water;
b) heating the suspension in order to dissolve completely the Ibandronate sodium.
   Preferably, the mixture is heated to a temperature comprised in a range of about 80 to about 100 °C;
c) Cooling the solution to a suitable temperature in order to crystallize a solid. A suitable temperature is comprised in a range of about 5 °C to about 25 °C; preferably, the temperature is comprised in a range of about 10 °C to about 20 °C; more preferably, the temperature is about 15 °C.
d) recovering the crystalline form.

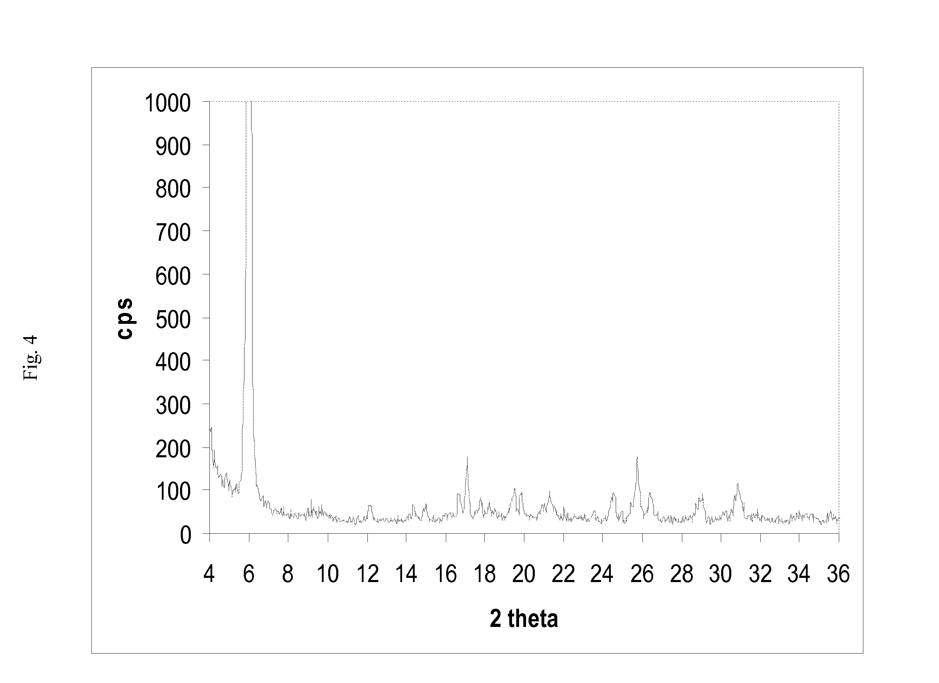

The novel crystalline anhydrous Form δ of Ibandronate sodium has an XRPD pattern as depicted in Figure 4.

In another aspect, the invention provides a process for the preparation of Form δ of Ibandronate sodium comprising the step of desolvating Ibandronate sodium Form β by heating under reduced pressure for a suitable period of time. Temperature of desolvation is preferably in a range comprised of about 130 °C to about 140 °C. Pressure of desolvation is preferably in a range comprised from about 5 mm Hg to about 20 mm Hg. A suitable period of time is from 5 hours to 10 hours.

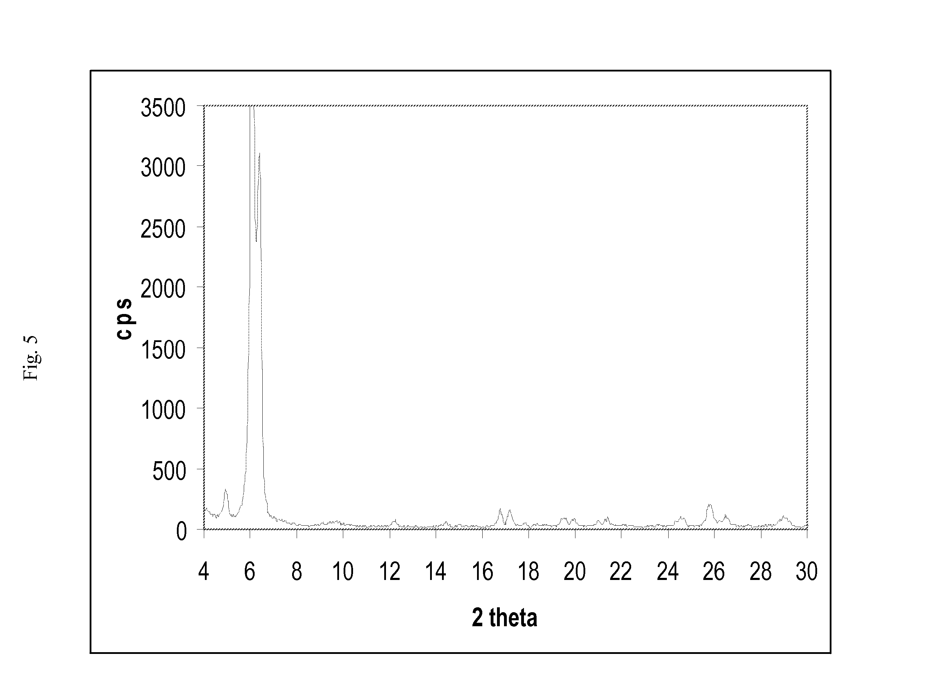

The novel crystalline monohydrate Form γ of Ibandronate sodium has an XRPD pattern as depicted in Figure 5.

In another aspect, the invention provides a process for the preparation of Form γ of Ibandronate sodium comprising the step of desolvating Ibandronate sodium Form ε by heating under reduced pressure for a suitable period of time. Temperature of desolvation is preferably in a range comprised of about 130 °C to about 140 °C. Pressure of desolvation is preferably in a range comprised from about 5 mm Hg to about 20 mm Hg. A suitable period of time is from 5 hours to 10 hours.

One skilled in the art will appreciate that by adjusting concentration, temperature and time the yield of crystalline Forms α, β, δ, ε, γ of Ibandronate sodium may be optimized.

As mentioned above the compound of the invention has useful therapeutic properties.

In another aspect, the invention provides crystalline Forms δ or γ for use as a medicament.

The Forms δ or γ of Ibandronate sodium may be administered per se or, preferably, as a pharmaceutical composition.

In another aspect, the invention provides a pharmaceutical composition comprising the crystalline Forms δ or γ, optionally together with at least one pharmaceutically acceptable excipient.
The polymorphic forms may be used as single components or mixtures.

Excipients include, by way of illustration and not limitation, diluents, fillers, agglutinants, disintegrants, disintegration inhibitors, absorption accelerators, binders, carriers, suspending/dispersing agents, film formers/coatings, adhesives, antiadherents, wetting agents, lubricants, glidants, preservatives, sorbents, surface active agents, substances added to mask or counteract a disagreeable taste or odor, flavorings, colorants, fragrances, aromatising agents, sweeteners and substances added to improve appearance of the composition.

A person skilled in the art is aware of a whole variety of such excipient compounds suitable to formulate a pharmaceutical composition. The choice of excipient will to a large extent depend on factors such as the particular mode of administration, the effect of the excipient on solubility and stability, and the nature of the dosage form.

A crystalline Form δ or γ of Ibandronate sodium, together with a conventionally employed excipient, may be placed into the form of pharmaceutical compositions and unit dosages thereof, and in such form may be employed as solids, such as tablets, capsules, pills, powders, granules, pellets, lozenges, pastilles, or liquids, such as solutions, suspensions, emulsions, drops, lotions, sprays, tinctures, syrups, elixirs, or capsules filled with the same, all for oral use, or in the form of sterile injectable solutions for parenteral (including subcutaneous, intramuscular, and intravenous) use, or in the form of suppositories for rectal use, or in the form of creams, ointments, gels, unguents for topical use and other forms suitable for the inhalatory or transdermal administrations. Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed.

Pharmaceutical compositions containing a crystalline Form δ or γ of Ibandronate sodium can be prepared in a manner well known in the pharmaceutical art and comprise at least one active compound. Generally, the compounds of this invention are administered in a pharmaceutically effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

The pharmaceutical compositions of the present invention can be administered by a variety of routes including oral, rectal, parenteral (including subcutaneous, intravenous, intramuscular), topical, transdermal, ophtalmic and intranasal. The compositions for oral administration can take the form of bulk liquid solutions or suspensions, or bulk powders. More commonly, however, the compositions are presented in unit dosage forms to facilitate accurate dosing. Typical unit dosage forms include prefilled, premeasured ampoules or syringes of the liquid compositions or pills, tablets, capsules, powders, granules, pellets, lozenges, pastilles, suppositories or the like in the case of solid compositions. In such compositions, a crystalline Form δ or γ of Ibandronate sodium is usually a minor component (from about 0.1 to about 50% by weight or preferably from about 1 to about 40% by weight) with the remainder being various vehicles or carriers and processing aids helpful for forming the desired dosing form.

Solid forms may include, for example, any of the following ingredients, or compounds of a similar nature: a binder, such as cellulose-derived materials like powdered cellulose, microcrystalline cellulose, microfine cellulose, methyl cellulose, ethyl cellulose, hydroxyethyl cellulose, hydroxypropyl cellulose, hydroxypropylmethyl cellulose, carboxymethyl cellulose salts and other substituted and unsubstituted celluloses, gum tragacanth or gelatine; an excipient, such as starch or lactose, a disintegrating agent, such as alginic acid, primogel, or corn starch; a lubricant, such as magnesium stearate, hydrogenated castor oil, polyethylene glycol; a glidant, such as colloidal silicon dioxide; a sweetening agent, such as sorbitol, sucrose, aspartame or saccharin; or a flavoring agent, such as maltol, vanillin, menthol, citric acid, pepper-mint, methyl salicylate, or orange flavoring.

Liquid forms suitable for oral administration may include a suitable aqueous or nonaqueous vehicle with buffers, suspending and dispersing agents, colorants, flavors and the like.

Injectable compositions are typically based upon injectable sterile saline or phosphate-buf- fered saline or other injectable carriers known in the art. As above mentioned, a crystalline Form δ or γ of Ibandronate sodium in such compositions is typically a minor component, with the remainder being the injectable carrier and the like.

The above described components for orally administered or injectable compositions are merely representative. Further materials as well as processing techniques and the like are set out in Part 5 of Remington 's Pharmaceutical Sciences, 20^{th} Edition, 2000, Merck Publishing Company, Easton, Pennsylvania, which is incorporated herein by reference. A crystalline Form δ or γ of Ibandronate sodium of this invention can also be administered in sustained release forms or from sustained release drug delivery systems. A description of representative sustained release materials can also be found in the incorporated materials in Remington's Pharmaceutical Sciences.

While the present invention has been described in terms of its specific embodiments, certain modifications and equivalents will be apparent to those skilled in the art and are included within the scope of the present invention.

In the following, the present invention shall be illustrated by means of some examples, which are not construed to be viewed as limiting the scope of the invention.

### Example 1

### Preparation of Form α of Ibandronate sodium

Ibandronate sodium (20 g) were charged at 20 °C in a round bottom flask containing water (24 ml). While stirring, the mixture was heated to 90°C. The hot solution was then poured in N-methyl-2-pirrolidinone (100 ml) kept at 15 °C in order for Ibandronate sodium Form α to precipitate. The resulting solid was filtered, washed with cold water and dried under reduced pressure at 60 °C. Ibandronate sodium Form α (9 g), having an XRPD pattern as substantially depicted in Figure 1, was obtained.

### Example 2

### Preparation of Form β of Ibandronate sodium

Ibandronate sodium (20 g) were charged at 20 °C in a round bottom flask containing water (24 ml). While stirring, the mixture was heated to 90°C. The hot solution was then poured in 2-propanol (100 ml), kept at 15 °C in order for Ibandronate sodium Form β to precipitate. The resulting solid was filtered, washed with cold 2-propanol and dried under reduced pressure at 60 °C. Ibandronate sodium Form β (15 g), having an XRPD pattern as substantially depicted in Figure 2, was obtained.

### Example 3

### Preparation of Form ε of Ibandronate sodium

Ibandronate sodium (20 g) was suspended at 20 °C in a round bottom flask containing water (100 mL) and 2-propanol (130 mL). While stirring, the mixture was heated to 85°C until a clear solution was obtained. Then the system was cooled to 15 °C and the resulting solid was filtered, washed with 2-propanol and dried under reduced pressure at 50 °C. Ibandronate sodium Form ε (19 g), having an XRPD pattern as substantially depicted in Figure 3, was obtained.

### Example 4

### Preparation of Form of Ibandronate sodium

Ibandronate sodium Form β was heated at 135 °C under a reduced pressure of 5 mm Hg. Desolvation process affords Ibandronate sodium Form δ, having an XRPD as substantially depicted respectively in Figure 4.

### Example 5

### Preparation of Form γ of Ibandronate sodium

Ibandronate sodium Form s was heated at 135 °C under a reduced pressure of 5 mm Hg. Desolvation process affords Ibandronate sodium Form γ, having an XRPD as substantially depicted respectively in Figure 5.

## Claims

1. A crystalline form of Ibandronate sodium (Form γ) having an X-ray powder diffraction pattern comprising peaks at about 4.9, 6.1, 6.5, 12.2, 16.8, 25.8 ± 0.2° 2θ.

2. A crystalline form according to claim 1, wherein said form is a monohydrate and is substantially **characterized by** an X-ray powder diffraction pattern in accordance with Figure 5.

3. A process for preparing the crystalline form according to claim 1, which comprises the step of desolvating crystalline form of Ibandronate sodium (Form ε) having an X-ray powder diffraction pattern comprising peaks at about 4.9, 6.0, 17.1, 19.8 ± 0.2° 2 θ, by heating the solid under reduced pressure.

4. The process according to claim 3, wherein the pressure of desolvation is preferably in a range comprised from about 5 mm Hg to about 20 mm Hg.

5. The process according to claim 3, wherein the temperature of desolvation is preferably in a range comprised from about 130 °C to about 140 °C.

6. A crystalline form according to claim 1 for use as a medicament.

7. A pharmaceutical composition comprising the crystalline form according to claim 1, optionally together with at least one pharmaceutically acceptable excipient.

8. A crystalline form of Ibandronate sodium (Form ε) having an X-ray powder diffraction pattern comprising peaks at about 4.9, 6.0, 17.1, 19.8 ± 0.2° 2 θ.

9. A crystalline form according to claim 8, substantially **characterized by** an X-ray powder diffraction pattern in accordance with Figure 3.

10. A crystalline form according to claims 8 or 9, wherein said crystalline form is a solvate of 2-propanol.

11. A process for preparing the crystalline form according to claim 8, which comprises the steps of:
a) suspending Ibandronate sodium in a mixture of water and 2-propanol;
b) heating the suspension in order to obtain a clear solution;
c) cooling the solution in order to crystallize a solid;
d) recovering the crystalline form.

12. The process according to claim 11, wherein in step a) water and 2-propanol are in a ratio of about 1:3 to about 1:5 volume/volume.

13. The process according to claim 11, wherein in step a) Ibandronate sodium is in a ratio of about 1:4 to about 1:6 weight/volume with to water.

14. The process according to claim 11, wherein in step b) the suspension is heated to a temperature comprised in a range of about 80 to about 100 °C.

15. A crystalline form of Ibandronate sodium (Form δ) having an X-ray powder diffraction pattern comprising peaks at about 5.8, 17.0, 25.6, 26.3, 30.8 ± 0.2° 2 θ.

16. A crystalline form according to claim 15, wherein said form is anhydrous and is substantially **characterized by** an X-ray powder diffraction pattern in accordance with Figure 4.

17. A process for preparing the crystalline form according to claim 15, which comprises the step of desolvating crystalline form of Ibandronate sodium (Form β) having an X-ray powder diffraction pattern comprising peaks at about 4.9, 9.6, 17.1, 19.8 ± 0.2° 2 θ, by heating the solid under reduced pressure.

18. The process according to claim 17, wherein the pressure of desolvation is preferably in a range comprised from about 5 mm Hg to about 20 mm Hg.

19. The process according to claim 17, wherein the temperature of desolvation is preferably in a range comprised from about 130 °C to about 140 °C.

20. A crystalline form according to claim 15 for use as a medicament.

21. A pharmaceutical composition comprising the crystalline form according to claim 15, optionally together with at least one pharmaceutically acceptable excipient.

22. A crystalline form of Ibandronate sodium (Form β) having an X-ray powder diffraction pattern comprising peaks at about 4.9, 9.6, 17.1, 19.8 ± 0.2° 2 θ.

23. A crystalline form according to claim 22, substantially **characterized by** an X-ray powder diffraction pattern in accordance with Figure 2.

24. A crystalline form according to claims 22 or 23, wherein said crystalline form is a solvate of2-propanol.

25. A process for preparing the crystalline form according to claim 22, which comprises the steps of:
a) dissolving Ibandronate sodium in water;
b) heating the solution;
c) pouring the solution in 2-propanol to obtain a precipitate;
d) recovering the crystalline form.

26. The process according to claim 25, step a), wherein the Ibandronate sodium is in a ratio of about 1:1 to about 1:1.5 weight/volume to water.

27. The process according to claim 25, step b), wherein the solution is heated to a temperature comprised in a range of about 80 to about 100 °C

28. A crystalline form of Ibandronate sodium (Form α) having an X-ray powder diffraction pattern comprising peaks at about 5.0, 10.0, 16.1, 19.8, 27.7 ± 0.2° 2 θ.

29. A crystalline form according to claim 28, substantially **characterized by** an X-ray powder diffraction pattern in accordance with Figure 1.

30. A crystalline form according to claims 28 or 29, wherein said crystalline form is a solvate of N-methyl-2-pyrrolidinone.

31. A process for preparing the crystalline form according to claim 28, which comprises the steps of:
a) dissolving Ibandronate sodium in water;
b) heating the solution;
c) pouring the solution in N-methyl-2-pyrrolidinone to obtain a precipitate;
d) recovering the crystalline form.

32. The process according to claim 31, step a), wherein the Ibandronate sodium is in a ratio of about 1:1 to about 1:1.5 weight/volume to water

33. The process according to claim 31, step b), wherein the solution is heated to a temperature comprised in a range of about 80 to about 100 °C.
